Europäisches Patentamt

European Patent Office

Office européen des brevets

(19)

(11) Veröffentlichungsnummer: **0 598 254 A1**

# (12) EUROPÄISCHE PATENTANMELDUNG

(21) Anmeldenummer: **93117377.7**

(22) Anmeldetag: **27.10.93**

(51) Int. Cl.5: **C10G 7/08**

(30) Priorität: **17.11.92 DE 4238716**

(43) Veröffentlichungstag der Anmeldung:
**25.05.94 Patentblatt 94/21**

(84) Benannte Vertragsstaaten:
**AT BE DE ES FR GB IT NL SE**

(71) Anmelder: **Krupp Koppers GmbH**
**Altendorfer Strasse 120**
**D-45143 Essen(DE)**

(72) Erfinder: **Firnhaber, Bernard, Dr.**
**Pelmanstrasse 40**
**D-45131 Essen(DE)**
Erfinder: **Emmrich, Gerd**
**Kamperfeld 21**
**D-45133 Essen(DE)**
Erfinder: **Kolbe, Bärbel, Dr.**
**Albertstrasse 14**
**D-58452 Witten(DE)**
Erfinder: **Verwey, Claudia**
**Altendorfer Strasse 352**
**D-45143 Essen(DE)**

(54) **Verfahren zur Trennung von Kohlenwasserstoffgemischen durch Extraktivdestillation.**

(57) Bei diesem Verfahren werden Lösungsmittel verwendet, die sich im Bezug auf die jeweilige Trennaufgabe durch eine hohe Selektivität auszeichnen und gleichzeitig mit den Kohlenwasserstoffen des Einsatzproduktes unter den angewandten Konzentrations- und Temperaturbedingungen Mischungslücken bilden. Die Stoffaustauschbedingungen in der Extraktivdestillationskolonne werden dabei so gestaltet, daß sowohl zwischen den beiden flüssigen Phasen untereinander als auch zwischen den flüssigen Phasen und der Dampfphase große Austauschflächen vorhanden sind.

EP 0 598 254 A1

Rank Xerox (UK) Business Services
(3.10/3.09/3.3.4)

Die Erfindung betrifft ein Verfahren zur Trennung von Kohlenwasserstoffgemischen durch Extraktivdestillation mit selektiven Lösungsmitteln bzw. Lösungsmittelgemischen, wobei das Einsatzprodukt in den mittleren Teil und das verwendete Lösungsmittel bzw. Lösungsmittelgemisch in den oberen Teil der Extraktivdestillationskolonne eingeleitet wird und die im Lösungsmittel-Kohlenwasserstoff-Gemisch leichter siedenden Kohlenwasserstoffe des Einsatzproduktes über Kopf aus der Extraktivdestillationskolonne abgezogen werden, während die schwerer siedenden Kohlenwasserstoffe des Einsatzproduktes zusammen mit der Hauptmenge des Lösungsmittels als Sumpfprodukt der Extraktivdestillation anfallen und das Sumpfprodukt aus der Extraktivdestillationskolonne in eine nachgeschaltete Abtriebskolonne überführt wird, in der die Kohlenwasserstoffe und das Lösungsmittel destillativ voneinander getrennt werden.

Extraktivdestillationen der vorstehend genannten Art werden heute in einer ganzen Reihe von großtechnischen Prozessen mit Erfolg eingesetzt, beispielsweise zur Gewinnung von Reinaromaten oder Butadien aus diese Verbindungen enthaltenden Einsatzprodukten sowie zur Trennung von Butanen und Butenen. Als besonders erfolgreiche Lösungsmittel haben sich dabei die N-substituierten Morpholine, insbesondere das N-Formylmorpholin, sowie N-Methylpyrrolidon und Dimethylformamid erwiesen. Bei allen Extraktivdestillationsverfahren wurden jedoch bisher die Prozeßbedingungen und das Lösungsmittel so gewählt, daß in der Extraktivdestillationskolonne nur eine Dampf- und eine Flüssigphase auftraten. Die Fachwelt ging dabei stets von der Überlegung aus, daß das Auftreten von Systemen mit Mischungslücken, bei denen sich in der Extraktivdestillationskolonne zwei flüssige Phasen ausbilden, vermieden werden muß. Diese Überlegungen wurden vor allem damit begründet, daß unter den in der Extraktivdestillation bisher üblichen Betriebsbedingungen das Vorhandensein einer zweiten, kohlenwasserstoffreichen Flüssigphase die Trennergebnisse wesentlich verschlechtert bzw. in manchen Fällen die Trennung sogar unmöglich macht. Die Gründe für die schlechte Trennung sind zum einen in den ungünstigen Stoffaustauschbedingungen zu suchen, denn die Einstellung des thermodynamischen Gleichgewichts zwischen zwei flüssigen Phasen ist üblicherweise erheblich schwieriger zu erreichen als zwischen einer dampfförmigen und einer flüssigen Phase. Zum anderen verschlechtern sich durch das Auftreten der Mischungslücke die thermodynamischen Bedingungen für die Trennung, beispielsweise durch geringere Änderung der Dampfdrücke oder die Bildung von Heteroazeotropen.

Aus den genannten Gründen hat man bisher Lösungsmittel, die sich zwar durch eine hohe Selektivität, das heißt einen großen Einfluß auf die relative Flüchtigkeit, für die zu trennenden Kohlenwasserstoffe auszeichnen, mit diesen aber unter den technisch interessanten Konzentrations- und/oder Temperaturverhältnissen zwei flüssige Phasen bilden, nicht für die Extraktivdestillation eingesetzt. Als Beispiel hierfür sei das unter dem Trivialnamen Sulfolan bekannte Tetramethylensulfon genannt, das lediglich in der Flüssig-Flüssig-Extraktion größere technische Bedeutung erlangt hat.

Der Erfindung liegt die Aufgabe zugrunde, ein Extraktivdestillationsverfahren zur Trennung von Kohlenwasserstoffgemischen zu schaffen, das sich auf an sich bekannten Anwendungsgebieten durch große wirtschaftliche Verbesserungen auszeichnet und das eine größere Lösungsmittelauswahl ermöglicht.

Das der Lösung dieser Aufgabe dienende Verfahren der eingangs genannten Art ist erfindungsgemäß gekennzeichnet durch die Anwendung der Merkmale a) bis c) des Hauptanspruches.

Die Erfindung geht dabei von der Erkenntnis aus, daß in Abkehr von den bisherigen Vorstellungen der Fachwelt Lösungsmittel, die sich für die jeweilige Trennaufgabe durch eine hohe Selektivität auszeichnen, aber gleichzeitig mit den Kohlenwasserstoffen des Einsatzproduktes unter den in der Praxis angewandten Konzentrations- und Temperaturbedingungen Mischungslücken bilden, sehr wohl in der Extraktivdestillation eingesetzt werden können, sofern in der Extraktivdestillation ganz bestimmte Bedingungen eingehalten werden.

Das erfindungsgemäße Verfahren gestattet deshalb insbesondere den Einsatz solcher Lösungsmittel, die sich für die jeweilige Trennaufgabe durch eine höhere Selektivität auszeichnen als die bisher eingesetzten Lösungsmittel, deren Anwendung aber wegen der Tendenz zur Bildung von Mischungslücken unterblieben ist.

Das erfindungsgemäße Verfahren gestattet aber auch den Einsatz solcher Lösungsmittel, deren Verwendung in der Extraktivdestillation für bestimmte Trennaufgaben bereits bekannt war. Hierbei kann dann mit wesentlich niedrigen Betriebstemperaturen in der Extraktivdestillation gearbeitet werden als dies bisher üblich war. Es hat sich nämlich bei vielen Lösungsmitteln gezeigt, daß die Tendenz zur Bildung von Mischungslücken nur bei relativ niedrigen Temperaturen auftritt. Wenn nun erfindungsgemäß trotzdem in diesem Temperaturbereich gearbeitet werden kann, so bedeutet dies eine nicht unbeträchtliche Energieeinsparung. Außerdem steigt die Selektivität des Lösungsmittels für die gegebene Trennaufgabe mit sinkender Betriebstemperatur.

Bei der Durchführung des erfindungsgemäßen Verfahrens müssen die Prozeßbedingungen in der Extraktivdestillation allerdings so gewählt werden, daß in einem erheblichen Teil der Gesamthöhe der Extraktivdestillationskolonne zwei flüssige Phasen auftreten. Vorzugsweise sollen hierbei zwischen 10 und 90 % der Kolonnenhöhe im Bereich der Zweiphasenbildung liegen und lediglich in dem restlichen unteren Teil der Kolonne soll nur eine homogene Flüssigphase zugegen sein.

Außerdem müssen die Stoffaustauschbedingungen in der Extraktivdestillationskolonne so gestaltet werden, daß sowohl zwischen den beiden flüssigen Phasen untereinander als auch zwischen den flüssigen Phasen und der Dampfphase große Austauschflächen vorhanden sind. Dies kann beispielsweise dadurch geschehen, daß in der Extraktivdestillationskolonne Einbauten bzw. Kolonnenpackungen vorgesehen werden, die die Bildung von sehr dünnen Flüssigkeitsschichten gewährleisten. Im technischen Maßstab wird man hierfür insbesondere geordnete Kolonnenpackungen, z.B. sogenannte strukturierte Packungen, wie zum Beispiel sogenannte Mellapack-Packungen, oder Streckmetall-Einbauten vorsehen. Ferner ist es möglich, durch den Einsatz von speziell dafür ausgelegten Kolonnenböden die geforderten Stoffaustauschbedingungen zu erfüllen. Diese Kolonnenböden sorgen für eine gute Verteilung der flüssigen Phasen ineinander bei gleichzeitig hoher Austauschfläche mit der Dampfphase. Beim Einsatz derartiger Kolonnenböden muß jedoch ein gewisser Druckabfall in der Extraktivdestillationskolonne in Kauf genommen werden. Schließlich besteht auch noch die Möglichkeit, durch mechanische Dispergierung der Flüssigphasen und des Dampfes, z.B. durch den Einsatz von Rührern, für den notwendigen Stoffaustausch zu sorgen.

Da bei der Durchführung des erfindungsgemäßen Verfahrens die hydraulischen Bedingungen in der Extraktivdestillationskolonne, wie Schichtdicke und Durchmischung der beiden flüssigen Phasen, eine entscheidende Rolle spielen, ist es zweckmäßig, daß die Durchsatzmenge von Einsatzprodukt und Lösungsmittel einen Wert von 200 m$^3$ pro m$^2$ Kolonnenquerschnitt und Stunde nicht überschreitet. Die maximale Durchsatzmenge ist dabei insbesondere abhängig von der Art der Kolonneneinbauten und der Viskosität des Lösungsmittels. Bei den hier beschriebenen Trennverfahren und Verwendung von strukturierten Kolonnenpackungen beträgt die maximale Durchsatzmenge ca. 50 m$^3$ pro m$^2$ Kolonnenquerschnitt und Stunde.

Da die erfindungsgemäß zur Anwendung gelangenden Lösungsmittel sich durch eine sehr hohe Selektivität und ein verhältnismäßig niedriges Lösevermögen auszeichnen, resultiert aus dem niedrigeren Lösevermögen eine größere Flüchtigkeit auch für die aus dem Sumpf der Extraktivdestillationskolonne auszutragende Komponente des Einsatzproduktes. In der Praxis kann deshalb die Sumpftemperatur in der Extraktivdestillationskolonne deutlich niedriger eingestellt werden als beim Einsatz von Lösungsmitteln mit höherem Lösevermögen. Daraus ergibt sich ein geringerer Wärmebedarf für die Extraktivdestillation, was gleichzeitig für die Beheizung des Kolonnensystems den Einsatz von Dampf geringerer Qualität, zum Beispiel Niederdruckdampf statt Mitteldruckdampf, ermöglicht.

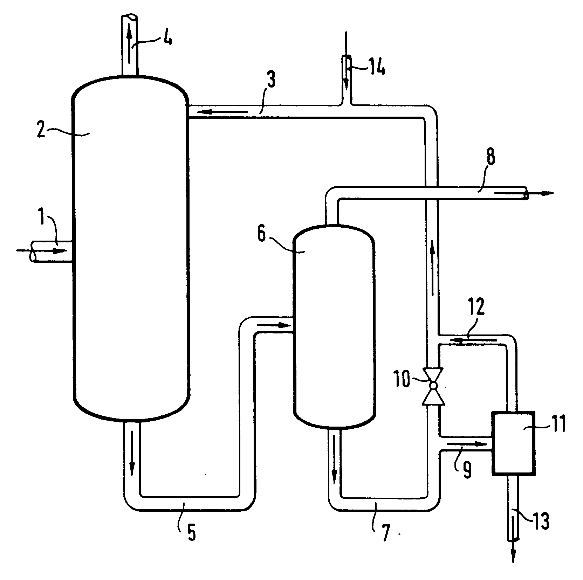

Für die Durchführung des erfindungsgemäßen Verfahrens können die für Extraktivdestillationen üblichen und allgemein gebräuchlichen Apparaturen und Anlagen eingesetzt werden. Der Verfahrensgang der erfindungsgemäßen Arbeitsweise ist dabei in dem in der Abbildung dargestellten Fließschema in vereinfachter Form wiedergegeben. Das aufzutrennende Kohlenwasserstoffgemisch wird hierbei durch die Leitung 1 im flüssigen Zustand in den mittleren Teil der Extraktivdestillationskolonne 2 eingeleitet, die mit den für die Herstellung der erforderlichen Stoffaustauschbedingungen notwendigen Einbauten versehen ist, wie dies weiter oben näher erläutert wurde. Durch die Leitung 3 wird das Lösungsmittel am Kopf in die Extraktivdestillationskolonne 2 eingeleitet und fließt über die Einbauten dieser Kolonne herab nach unten, wobei es die schwerer flüchtigen Kohlenwasserstoffe aufnimmt. Die leichter flüchtigen Kohlenwasserstoffe entweichen durch Leitung 4 am Kopf der Kolonne und können in im Fließschema nicht dargestellten Kondensationseinrichtungen kondensiert werden. Das flüssige Sumpfprodukt besteht aus dem Lösungsmittel und den darin gelösten Kohlenwasserstoffen und wird durch die Leitung 5 aus der Extraktivdestillationskolonne 2 abgezogen und gelangt in die Abtriebskolonne 6, in der die Kohlenwasserstoffe destillativ vom Lösungsmittel abgetrennt werden. Das Lösungsmittel wird durch die Leitung 7 aus dem Kolonnensumpf entfernt und gelangt nach entsprechender Abkühlung, die im allgemeinen in einem wirtschaftlichen Wärmeverbund innerhalb der Anlage erreicht wird, über die Leitung 3 wieder in die Extraktivdestillationskolonne 2 zurück, während die Kohlenwasserstoffdämpfe über Kopf aus der Abtriebskolonne 6 entweichen und durch die Leitung 8 ihrer weiteren Verarbeitung zugeführt werden. Da sich im Laufe der Zeit im Lösungsmittel Verunreinigungen anreichern können, ist im Bereich der Leitung 7 die Abzweigleitung 9 vorgesehen, durch die bei entsprechender Stellung des Ventils 10 eine Teilmenge des Lösungsmittels zur Regenerier-Einrichtung 11 gelangen kann. Das regenerierte Lösungsmittel wird durch die Leitung 12 wieder in den Kreislauf (Leitung 7) zurückgeführt, während die ausgeschiedenen Verunreinigungen durch die Leitung 13 aus der Regeneriereinrichtung 11 abgezogen werden. Die Leitung 14 dient schließlich der Zufuhr von frischem Lösungsmittel. Selbstverständlich ist die Anlage zur Durchführung des erfindungsgemäßen Verfahrens mit den erforderlichen Nebeneinrichtungen, wie z.B. Meß- und Regeleinrichtungen sowie Wärmetauschern, ausgerüstet. Da jedoch diese Nebeneinrichtungen zur Erläuterung des Verfahrensablaufes nicht unmittelbar erforderlich sind, wurden sie in der Abbildung nicht dargestellt.

Das erfindungsgemäße Verfahren kann zur Lösung unterschiedlicher Trennaufgaben eingesetzt werden. So kann beispielsweise das Verfahren zur Gewinnung von Reinaromaten aus aromatenhaltigen Kohlenwasserstoffen herangezogen werden. Hierbei können Sulfone oder deren Gemische, N-substituierte Morpholine oder deren Gemische sowie vorzugsweise ein Gemisch aus N-Formylmorpholin und Tetramethylensulfon (Sulfolan) als Lösungsmittel verwendet werden. Die Verwendung von N-substituierten Morpholinen oder deren Gemischen ist zwar für die genannte Trennaufgabe bereits bekannt. Das erfindungsgemäße Verfahren unterscheidet sich dabei jedoch von der bisher üblichen Arbeitsweise durch die Anwendung einer niedrigeren, zwischen 50 und 70 ° C liegenden Lösungsmittel-Aufgabetemperatur in der Extraktivdestillation. Die Lösungsmittel-Aufgabetemperatur liegt in diesem Falle ca. 10 bis 30 ° C niedriger als bei der bekannten Arbeitsweise mit homogener Flüssigphase. Ein weiteres Anwendungsgebiet des erfindungsgemäßen Verfahrens ist die Trennung von Diolefinen, Olefinen und Paraffinen aus diese Verbindungen enthaltenden, bei Normaldruck im Bereich zwischen -12 und +50 ° C siedenden Kohlenwasserstoffgemischen unter Verwendung von Sulfonen oder deren Gemischen als Lösungsmittel. Schließlich kann das erfindungsgemäße Verfahren auch zur Trennung von Olefinen und Paraffinen aus diese Verbindungen enthaltenden, bei Normaldruck im Bereich zwischen -12 und +50 ° C siedenden Kohlenwaserstoffgemischen eingesetzt werden, wobei wiederum N-substituierte Morpholine oder deren Gemische als Lösungsmittel zur Anwendung gelangen können. Die Verwendung dieser Lösungsmittel für diese Trennaufgabe ist zwar ebenfalls bereits bekannt, das erfindungsgemäße Verfahren unterscheidet sich jedoch von der bisher üblichen Arbeitsweise wiederum durch die Anwendung einer niedrigeren, zwischen 40 und 70 ° C liegenden Lösungsmittel-Aufgabetemperatur in der Extraktivdestillation. Auch hier liegt die Lösungsmittel-Aufgabetemperatur um ca. 10 bis 30 ° C niedriger als bei der bekannten Arbeitsweise mit homogener Flüssigphase.

Durch die niedrigere Lösungsmittel-Aufgabetemperatur wird jeweils der interne Rückfluß in der Extraktivdestillationskolonne erhöht. Dadurch wird bei einem geringeren Lösungsmittel : Einsatzprodukt-Verhältnis die gleiche Trennleistung wie bisher erzielt, gleichzeitig aber auch die Ausbildung von zwei flüssigen Phasen in der Extraktivdestillationskolonne erreicht.

Die Wirksamkeit des erfindungsgemäßen Verfahrens wird durch die beiden nachfolgend beschriebenen Vergleichsversuche belegt:

Der erste Versuch (Tabelle 1 ) betrifft dabei die Trennung von Butanen und Butenen durch Extraktivdestillation, wobei als Einsatzprodukt ein C$_4$-Schnitt aus Pyrolysebenzin verwendet wurde, der zuvor eine Butadienextraktion sowie unter Entfernung von 2-Methylpropen eine Veretherung mit Methanol zu Methyltert.-Butylether durchlaufen hatte. Im Teil 1 dieses Versuches wurde dabei ein Lösungsmittelgemisch verwendet, das zu 50 % aus N-Formylmorpholin (NFM) und zu 50 % aus Morpholin als Lösungsvermittler bestand. In Teil 2 gelangte dagegen als Lösungsmittel reines N-Formylmorpholin (NFM) zur Anwendung.

Der zweite Vergleichsversuch (Tabelle 2) betrifft die Gewinnung von Reinaromaten durch Extraktivdestillation aus einem Gemisch aus Pyrolysebenzin und katalytischem Reformat. Hierbei wurde in Teil 1 des Versuches N-Formylmorpholin (NFM) sowie in Teil 2 und 3 Tetramethylensulfon (Sulfolan) als Lösungsmittel verwendet. Im Teil 3 wurde dabei mit einem geringeren Lösungsmittel : Einsatzprodukt-Verhältnis gearbeitet als im Teil 2. Dies führt zu einem geringeren Wärmebedarf, wobei jedoch gleichzeitig die Reinheit des gewonnenen Benzols etwas niedriger ausfällt.

Beide Vergleichsversuche wurden in einer Extraktivdestillationsanlage der weiter oben im Zusammenhang mit der Abbildung beschriebenen Art durchgeführt. Im Teil 2 bzw. 3 der Versuche, bei dem es zur Bildung von zwei flüssigen Phasen in der Extraktivdestillation kommt, waren in der Extraktivdestillationskolonne strukturierte Packungen aus Streckmetall angeordnet. Gleichzeitig wurde für Teil 2 bzw. 3 die Höhe der Extraktivdestillationskolonne um etwa 30 % reduziert. Die Versuchsbedingungen wurden bei beiden Vergleichsversuchen jeweils über 72 Stunden konstant gehalten. Die wesentlichen Versuchsbedingungen und Trennergebnisse sind nachfolgend in den Tabellen 1 und 2 zusammengefaßt.

4

Tabelle 1: Trennung von Butanen und Butenen durch
Extraktivdestillation

| Lösungsmittel (LM) | | 50 % NFM/50 % Lö-sungsvermittler | NFM |
|---|---|---|---|
| LM/Einsatz | kg/kg | 17 | 17 |
| Durchsatz ED | kg/h | | |
| -Konzentration (LM-frei) | | | |
| - Kopf der ED | | | |
| iso-Butan | Gew.% | 23,72 | 23,72 |
| n-Butan | Gew.% | 73,32 | 73,79 |
| 1-Buten | Gew.% | 2,94 | 2,41 |
| trans-2-Buten | Gew.% | 0,02 | 0,054 |
| cis-2-Buten | Gew.% | 0,0005 | 0,018 |
| - Einsatz | | | |
| Iso-Butan | Gew.% | 6,84 | 6,84 |
| n-Butan | Gew.% | 22,44 | 22,44 |
| 1-Buten | Gew.% | 43,04 | 43,04 |
| trans-2-Buten | Gew.% | 11,97 | 11,97 |
| cis-2-Buten | Gew.% | 15,71 | 15,71 |
| - Sumpf der ED | | | |
| iso-Butan | Gew.% | - | - |
| n-Butan | Gew.% | 1,85 | 1,67 |
| 1-Buten | Gew.% | 59,27 | 59,47 |
| trans-2-Buten | Gew.% | 22,06 | 22,04 |
| cis-2-Buten | Gew.% | 16,82 | 16,81 |
| Betriebstemperatur | | | |
| ED-Sumpf | °C | 154 | 97,4 |
| Betriebsdruck | bar | 5,9 | 5,9 |

5

```
Höhe der homogenen
Flüssigphase in der ED      %              100            22
------------------------------------------------------------

Wärmebedarf pro
Einsatz                  kcal/kg         1199  .          563
------------------------------------------------------------
```

Tabelle 2

| Erzeugung von Reinbenzol durch Extraktivdestillation | | | | | |
|---|---|---|---|---|---|
| Lösungsmittel (LM) | | | NFM | Sulfolan | Sulfolan |
| LM/Einsatz | kg/kg | | 2,5 | 2,5 | 2,2 |
| Durchsatz ED | kg/h | | | | |
| Konzentration (LM-frei) | | | | | |
| - Kopf der ED | | | | | |
| Nichtaromaten | Gew.% | | 97,20 | 97,12 | 97,20 |
| Methylcyclohexan | Gew.% | | 0,54 | 0,62 | 0,54 |
| Benzol | Gew.% | | 2,26 | 2,26 | 2,26 |
| - Einsatz | | | | | |
| Nichtaromaten | Gew.% | | 46,1 | 46,1 | 46,1 |
| Methylcyclohexan | Gew.% | | 0,3 | 0,3 | 0,3 |
| Benzol | Gew.% | | 53,6 | 53,6 | 53,6 |
| - Sumpf der ED | | | | | |
| Nichtaromaten | ppm | | - | - | - |
| Methylcyclohexan | ppm | | 795 | 94 | 788 |
| Benzol | Gew.% | | 99,92 | 99,99 | 99,92 |
| Betriebstemperatur ED-Sumpf | °C | | 156 | 158 | 151 |
| Betriebsdruck | bar | | 2,3 | 2,3 | 2,3 |
| Höhe der homogenen Flüssigphase in der ED | % | | 100 | 80 | 60 |
| Wärmebedarf pro Einsatz | kcal/kg | | 152 | 133 | 118 |

Die vorliegenden Versuchsergebnisse zeigen, daß bei Anwendung des erfindungsgemäßen Verfahrens eine deutliche Verringerung des Wärmebedarfs pro Einsatz erreicht wird, wobei im Vergleichsversuch 1 fast eine Halbierung des Energieverbrauches auftrat. Hinzu kommt außerdem noch die Reduzierung der Anlagekosten, die sich durch die Verringerung der Höhe der Extraktivdestillationskolonne ergibt. Das angestrebte Ziel einer verbesserten Wirtschaftlichkeit der Extraktivdestillation wird somit erreicht.

**Patentansprüche**

1. Verfahren zur Trennung von Kohlenwasserstoffgemischen durch Extraktivdestillation mit selektiven Lösungsmitteln bzw. Lösungsmittelgemischen, wobei das Einsatzprodukt in den mittleren Teil und das verwendete Lösungsmittel bzw. Lösungsmittelgemisch in den oberen Teil der Extraktivdestillationskolonne eingeleitet wird und die im Lösungsmittel-Kohlenwasserstoff-Gemisch leichter siedenden Kohlenwasserstoffe des Einsatzproduktes über Kopf aus der Extraktivdestillationskolonne abgezogen werden, während die schwerer siedenden Kohlenwasserstoffe des Einsatzproduktes zusammen mit der Haupt-

6

menge des Lösungsmittels als Sumpfprodukt der Extraktivdestillation anfallen und das Sumpfprodukt aus der Extraktivdestillationskolonne in eine nachgeschaltete Abtriebskolonne überführt wird, in der die Kohlenwasserstoffe und das Lösungsmittel destillativ voneinander getrennt werden, **dadurch gekennzeichnet**, daß

a) Lösungsmittel verwendet werden, die sich im Bezug auf die jeweilige Trennaufgabe durch eine hohe Selektivität auszeichnen und die gleichzeitig mit den Kohlenwasserstoffen des Einsatzproduktes unter den angewandten Konzentrations- und Temperaturbedingungen Mischungslücken bilden,

b) die Prozeßbedingungen der Extraktivdestillation so gewählt werden, daß in einem erheblichen Teil der Gesamthöhe der Extraktivdestillationskolonne zwei flüssige Phasen auftreten und

c) die Stoffaustauschbedingungen in der Extraktivdestillationskolonne so gestaltet werden, daß sowohl zwischen den beiden flüssigen Phasen untereinander als auch zwischen den flüssigen Phasen und der Dampfphase große Austauschflächen vorhanden sind.

2. Verfahren nach Anspruch 1 , **dadurch gekennzeichnet**, daß die Betriebsbedingungen so eingestellt werden, daß zwischen 10 und 90% der Kolonnenhöhe der Extraktivdestillationskolonne im Bereich der Ausbildung von zwei flüssigen Phasen liegen und daß nur im restlichen unteren Teil der Extraktivdestillationskolonne eine homogene Flüssigphase vorliegt.

3. Verfahren nach den Ansprüchen 1 und 2, **dadurch gekennzeichnet**, daß die erforderlichen Stoffaustauschbedingungen in der Extraktivdestillationskolonne durch die Anordnung von Einbauten oder Kolonnenpakkungen in der Kolonne sichergestellt werden, die entweder die Ausbildung von sehr dünnen Flüssigkeitsschichten gewährleisten oder in anderer Weise für eine gute Durchmischung und Verteilung der einzelnen Phasen sorgen.

4. Verfahren nach den Ansprüchen 1 und 2, **dadurch gekennzeichnet**, daß die erforderlichen Stoffaustauschbedingungen in der Extraktivdestillationskolonne durch die Anwendung eines Rührers oder einer anderen mechanischen Dispergiereinrichtung in der Kolonne sichergestellt werden.

5. Verfahren nach den Ansprüchen 1 bis 4, **dadurch gekennzeichnet**, daß die Durchsatzmenge von Einsatzprodukt und Lösungsmittel in der Extraktivdestillationskolonne den Wert von 200 m$^3$ , vorzugsweise 50 m$^3$ , pro m$^2$ Kolonnenquerschnitt und Stunde nicht überschreitet.

6. Anwendung des Verfahrens nach den Ansprüchen 1 bis 5 zur Gewinnung von Reinaromaten aus aromatenhaltigen Kohlenwasserstoffgemischen, **dadurch gekennzeichnet**, daß Sulfone oder deren Gemische als Lösungsmittel verwendet werden.

7. Anwendung des Verfahrens nach den Ansprüchen 1 bis 5 zur Gewinnung von Reinaromaten aus aromatenhaltigen Kohlenwasserstoffgemischen unter Verwendung von N-substituierten Morpholinen oder deren Gemischen als Lösungsmittel, **dadurch gekennzeichnet,** daß die Extraktivdestillation bei einer Lösungsmittel-Aufgabetemperatur zwischen 50 und 70°C durchgeführt wird.

8. Anwendung des Verfahrens nach den Ansprüchen 1 bis 5 zur Gewinnung von Reinaromaten aus aromatenhaltigen Kohlenwasserstoffgemischen, **dadurch gekennzeichnet**, daß als Lösungsmittel ein Gemisch aus N-Formylmorpholin und Tetramethylensulfon (Sulfolan) verwendet wird.

9. Anwendung des Verfahrens nach den Ansprüchen 1 bis 5 zur Trennung von Diolefinen, Olefinen und Paraffinen aus diese Verbindungen enthaltenden, im Bereich zwischen -12 und +50°C siedenden Kohlenwasserstoffgemischen, **dadurch gekennzeichnet**, daß Sulfone oder deren Gemische als Lösungsmittel verwendet werden.

10. Anwendung des Verfahrens nach den Ansprüchen 1 bis 5 zur Trennung von Olefinen und Paraffinen aus diese Verbindungen enthaltenden, im Bereich zwischen -12 und +50°C siedenden Kohlenwasserstoffgemischen unter Verwendung von N-substituierten Morpholinen oder deren Gemischen als Lösungsmittel, **dadurch gekennzeichnet**, daß die Extraktivdestillation bei einer Lösungsmittel-Aufgabetemperatur zwischen 40 und 70°C durchgeführt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | US-A-4 053 369 (PHILLIPS PETROLEUM) <br> * Ansprüche 1-3 * <br> ----- | 1,2,6 | C10G7/08 |

| | RECHERCHIERTE SACHGEBIETE (Int.Cl.5) |
|---|---|
| | C10G <br> B01D <br> C07C |

Der vorliegende Recherchenbericht wurde für alle Patentansprüche erstellt

| Recherchenort | Abschlußdatum der Recherche | Prüfer |
|---|---|---|
| DEN HAAG | 9. Februar 1994 | De Herdt, O |